# EUROPEAN PATENT APPLICATION

(11) **EP 1 684 233 A1**
(43) Date of publication of application: **26.07.2006**
(21) Application number: 06250290.1
(22) Date of filing: 19.01.2006
(51) Int. Cl.: G06T 5/00

(54) **Method and apparatus for the preparation of a renal image**

(30) Priority: 25.01.2005 FR 0550218
(71) Applicant: The General Electric Company, Schenectady NY 12345 (US)
(72) Inventor: Ferrant, Matthieu Denis, Saint Remy Les Chevreuse (FR); Coric, Claude Eugene, 92000 Nanterre (FR); Knoplioch, Jérôme, 92200 Neuilly sur Seine (FR); Treutenaere, Jean-Marc, 60200 Compiegne (FR)
(74) Representative: Goode, Ian Roy

(57) **Abstract**

In an image of lithiasis in a computed tomography examination, a segment (34) of the image is chosen with a detection of the maximum of the gradient (30) of radiological density measured in the voxels of the reconstructed image. This mode of action gives an evaluation of the envelope of the lithiasis (29) that is objective and independent of the practitioner who performs it. Furthermore, to make the characterization of the lithiasis more realistic, especially to follow the effects of a therapeutic treatment, a characteristic result is given, equivalent to the volume of the lithiasis, preferably a weighted volume, and to the mean density of the lithiasis. The disclosed method also can facilitate the scheduling of surgical operations when it is not possible to administer oral treatment or carry out lithotripsy

## Description

This invention relates to the preparation of a renal image, and in particular, the preparation of a renal image by the use of computed tomography scanner. The field of the invention is that of medicine. An embodiment of the invention is to facilitate the detection of the presence of lithiasis or calcification also known as calculi or stones, situated in the kidneys, urinary apparatus (bladder, urethra etc.) or gall bladder of an object body such as a human patient. An embodiment of the invention also enables the appropriate treatment of lithiasis and, in the case of a surgical operation or procedure, to provide tools for planning the operation through computed tomography. An embodiment of the invention is also to enable the measurement of the efficiency of treatment being undergone by the object body, during this treatment. Further, an embodiment of the invention facilitates the scheduling of operations using surgical tools when it is not possible to administer oral treatment or practice lithotripsy.

In the diagnosis of renal affections, echography type examinations have hitherto been used because kidney stones respond well to an ultrasonic signal received. Furthermore, in radiology, there is the known practice of performing urography tests. During these urography tests, a contrast medium is injected by urinary channels into an object's body urinary tract and its path is followed on radiological images. Recently, however, attempts have been made to examine the kidney by means of computed tomography with significant results. Indeed, kidney stones show a marked differentiation in the renal images and enable precise densitometric and geometrical 3D quantification and localization.

However, the fine-tuning and perfecting of these processes for the acquisition of images of renal affections raise difficulties that are hard to surmount. In practice, unprocessed images are shown on the screen and, to enable a better interpretation, operator-assisted processes for the expansion of contrast and/or segmentation are implemented. This means that a practitioner must point to a portion of interest on the image. This portion of interest is highlighted by the practitioner. In this portion of interest, development processing operations are undertaken as a function of local parameters, which are themselves, also set by the practitioner. The reason for this input by the practitioner is as follows: It can be assumed that, on an axis that crosses the kidney and goes through a lithiasis, the radiological density signal, measured in Hounsfield units, HU, undergoes high growth. For example, its value may typically pass from 300 (corresponding to the density of bone) to 1000, especially when the calculus or stone is very dense. Normally, it would be possible to set a threshold at an intermediate value between these two values to discriminate or segment the image into two parts, namely the part that corresponds to the healthy kidney (with values of radiological density below the threshold) and the part that corresponds to the lithiasis (with higher values).

However, the stones are not always equally dense. Some are only in the process of being formed. In addition, the presence of the tissues surrounding the kidney has the effect of shifting the entire measured signal upward or downward. Hence, the presence of a fixed threshold of this kind may cause the segmentation to be falsified. This is why, in an image that is to some extent prepared, the practitioner is asked to set the threshold parameters locally. In practice, this operation must be performed section by section and, for each of the sections, it is necessary to determine the threshold from which the choice is made. For sufficient depth of examination, about 30 sections have to be processed (or more than 30 sections depending on the modalities of acquisition and the size of the lesions to be analyzed). This makes the job painstaking and furthermore not easily reproducible. As a consequence, this action by the practitioner is a drawback of the method.

Furthermore, if lithiasis is diagnosed, it is expected that the object body will be treated in order to be cured of this affection. The treatment may be oral and the object body will take medicines for some time, for example for several weeks. Otherwise, the treatment may also comprise sessions of lithotripsy during which the stone is bombarded with ultrasonic beams so that it may be destroyed by mechanical effect through the ultrasound that it receives. At the end of this phase of treatment, the object body again undergoes an examination of the same type as the first one. The aim than is to compare the images obtained during the first examination with the images obtained during a second examination. Given the fairly complex operation that is required of the practitioner, it will be very difficult to ask him or her to reprocess the images with exactly the same parameters as earlier. Since this is not done, the images are not really comparable and it is difficult to get an exact picture of the efficiency of the processing and that has been applied in the meantime.

An embodiment of the invention overcomes this problem that is essentially related to the action of the practitioner to edit the image. An embodiment of the invention provides a method that is entirely objective and not dependent on the practitioner's action.

In an embodiment of the invention, these problems are overcome by a method of segmentation in which, rather than determining that the voxels of the image belong to the lithiasis on the basis of a test relative to a threshold, these voxels are chosen by measuring the gradient of the radiological density, it being understood that at the position of the wall of the lithiasis, the value of the radiological density evolves greatly. Since, in lithiasis, it has been observed that the radiological density evolves less sharply, choose the position of the voxels for which the gradient of the radiological density is the maximum as being the position of the wall of the lithiasis. Consequently, the position of the maximum gradients is identified. In one embodiment, this identification is done by the implementation of a watershed algorithm, the LPE algorithm.

This mode of action leads to the observation that a contour, an envelope of the lithiasis, is determined independently of the operation by an operator: the measurement becomes objective and no longer subjective. Since the algorithmic processing, especially of the LPE type, is automatic, the determining of the lithiasis is thus automatic. The result of this, for the comparison of images acquired at different dates, at intervals of some weeks, is a reproducibility of the method that makes it reliable.

An embodiment of the invention is a method for the preparation of a renal image wherein an object body with a kidney is subjected to a sequence of irradiation by X-rays at various angles. Images in projection are taken at each of these angles. A first 3D image formed by voxels is produced from the images in projection, each voxel being assigned a measurement of the radiological density at its position, this image representing the body in and around the kidney. A seed voxel is chosen. From this seed voxel, the neighboring voxels in the 3D image are explored. For all the explored voxels, a gradient of the radiological density is measured. Those voxels located on an envelope for which the gradient is the maximum are selected and thus an envelope surface of a renal affection, logically a lithiasis, is obtained. A second image representing this envelope is produced and visualized.

An embodiment of the invention will be understood more clearly from the following description and the accompanying figures. These figures are given purely by way of an indication and in no way restrict the scope of the invention. Of these figures:
Figure 1 is a diagrammatic view of one embodiment of an apparatus that can be used to implement an embodiment of the method;
Figure 2 comprises two graphs showing, firstly, the progress of a signal of radiological density and secondly, the progress of the gradient of this signal with its consequence on the determining of the position of the wall of the lithiasis;
Figure 3 shows an improvement of the characterization of the assessment of the lithiasis in order to propose an objective measurement, to the practitioner, of its volume, weight, and density in terms of HU units as well as other statistical variables; and
Figure 4 shows the nature of the measured affection that can be brought out on a color image.

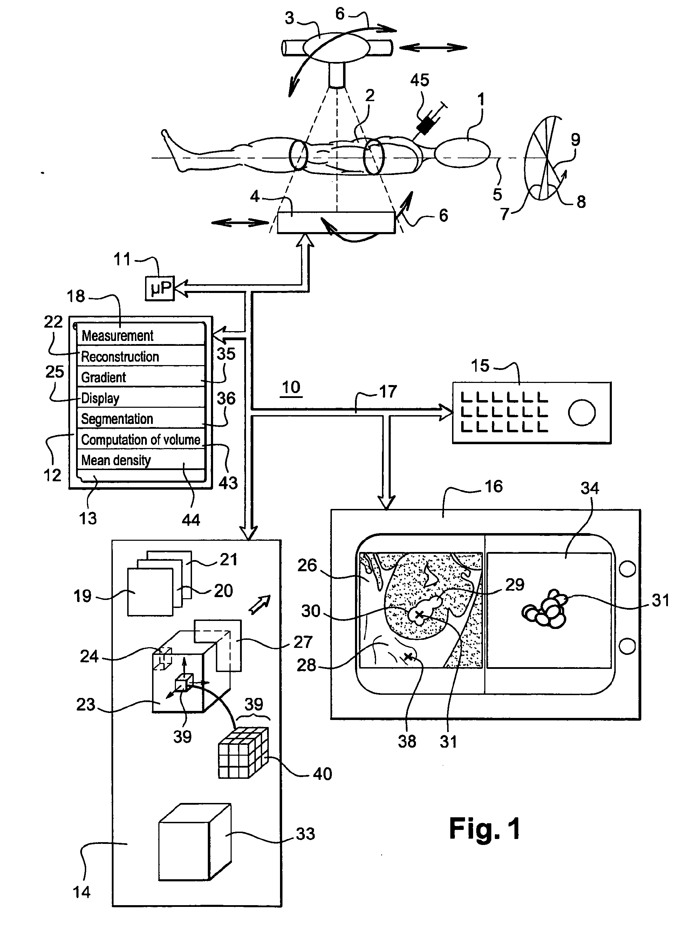

Figure 1 shows an example of a general apparatus that can be used to implement an embodiment of the method of the invention. In this method and apparatus, an object body 1 is put through a radiology examination. The object body can be a human being with a kidney located in a region 2 of the body. The region 2 of the body is the region examined. For example, the examination apparatus comprises a computed tomography scanner provided schematically with an X-ray tube 3 and a detector 4 together moved about an axis of rotation 5 in a step-by-step or continuous rotational motion 6. During this rotation, the region 2 is subjected to an irradiation sequence with X-rays at various angles, for example the angles such as 7 to 9. In computer tomography, for a given precision of measurement, there are constraints on the angular pitch of the different values of incidence at which the body is irradiated, as well as the minimum value of angular exploration (normally 180°). The apparatus thus described is driven by a computer system 10 comprising a microprocessor 11, a program memory 12 containing a program 13, a data memory 14, and control (15) peripherals and display (16) peripherals. In a known way, all these elements are connected to each other by a data, address and control bus 17. The different elements may furthermore have different and varied natures and shapes.

In program 13, a measurement sub-program 18 makes it possible, for each of the incidence values 7 to 9, to take projected images such as 19 to 21. The signals of these images are digitized and stored in the memory 14. In a known way, the program 13 furthermore has a reconstruction sub-program 22 with which, starting from the projected images 19 to 21, a digital volume 23 representing the region 2 is computed. The term "digital volume" is understood to mean a set of voxels such as 24, virtually geometrically assembled to form the volume 23, and containing a piece of information representing the radiological density, especially in terms of standardized HU units, of the position corresponding to them in the region 2 of the body 1. In the memory 14, the voxels are stored at addresses corresponding to their place in the region 2.

There also exist known display sub-programs 25 with which it is possible to represent, on the screen 16, sectional views of the digital volume 23, or even 3D views. The left-hand side of the screen 16 thus shows a view 26 of the affection 27 of the digital volume 23. In the prior art, using associated tools, the practitioner should get an idea, in the view 26, of a level of radiological density in a region 28 surrounding a region 29 in which a lithiasis is located. It should fix a segmentation threshold so as to enable the acquisition of contour 30, in the section 27, of the lithiasis 29. And so on and so forth for all the affections of the volume 23. It is this painstaking and hardly reproducible operation that is now essentially eliminated or reduced by an embodiment of the invention.

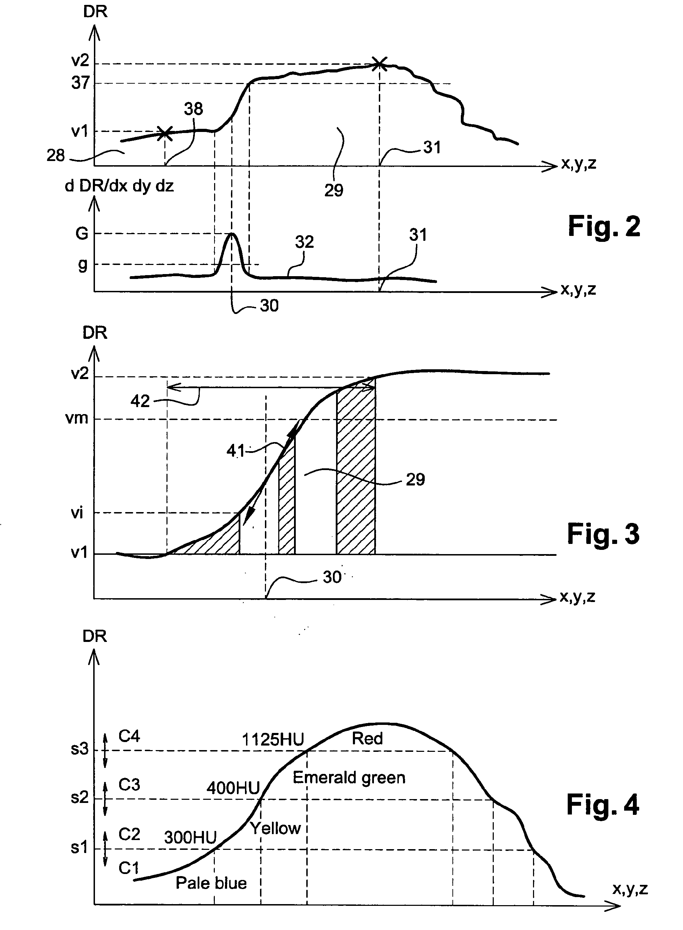

Figure 2 gives a view, as a function of an exploration of the digital volume, or of the region 2, which amounts to the same thing, in a symbolic direction x, y, z, of the levels of radiological density DR measured at the position of the lithiasis 29 and at the position 28 of the parts that surround it. It can be seen that, from one region to another, the level of radiological density evolves remarkably. The second graph of Figure 2 shows a view of the gradient of the radiological density signal DR as a function of the spatial coordinates. This gradient culminates in a value G right on 30 of the position of the contour. More specifically, the contour 30 is fixed right on the maximum of the gradient. At the practical level, the search for the gradient is made neither in a 1D image nor in a 2D image, but in the 3D image. A search is thus made in the digital volume 23 for the envelope of the peaks of the gradient, the envelope being the set of locations 30 of the wall of the lithiasis. In one example, the search algorithm is of the watershed type, namely the LPE algorithm, described for example in EP-A-0 576 584, the signal studied being the gradient signal. Otherwise, it could be of the K-Means segmentation, K-Nearest Neighbor segmentation, or Principal components analysis type, etc.

The segmentation of the volume image 23 to extract the sub-image corresponding to the lithiasis 29 therefore includes the computation of the gradient of the radiological density. Rather than having to perform this computation for all the voxels of the volume 23, it may be preferred to limit the computation to a certain number of them, especially the voxels of the lithiasis only. In practice, in this case, to arrive at the result more quickly, it is seen to it that the computation starts with a seed voxel, for example the voxel 31 pointed at by the practitioner in the middle of the lithiasis 29 in the display 26 of the section 27. If it is desired to do without this pointing operation, it is of course possible to make the computation of the gradient start with an arbitrary pixel, for example a voxel 24 located at one of the ends of the digital volume 23.

Starting with the voxels 31, typically situated to the right in the graph of Figure 2, the voxels neighboring this voxel 31 in the 3D image 23 are explored. For all the voxels encountered, of which there are initially 26, the gradient of the radiological density is measured. Continuing in this way, from one voxel to the next, the gradient values represented by the curve 32 of the second graph of Figure 2 are measured. Each of these gradient values may be stored in another digital image 33 (Figure 1) and assigned to a voxel of the image 33 corresponding to the volume voxel of the digital volume 23. In this way, the volume image 33 is the volume image of the gradient of the radiological density. As the case may be, at each address of the memory 14 corresponding to the storage of information on the voxels of the volume 23, it is possible to add attributes, one of these attributes being the gradient of the voxel concerned.

In the image 33, or in the image 23 with attributes, it is thus possible, in a known way, especially with the LPE type algorithm referred to here above, to produce a second image representing the envelope of the lithiasis. The envelope can thus be displayed as shown in the right-hand part of the screen 16, by the view 34. The view may be a view in section (like the view 26) or a 3D view that implements a corresponding display algorithm. In these conditions, the program 13 therefore has a sub-program 35 for the preparation of the gradient image 33 and a sub-program 36 for segmentation in this gradient image, the sub-program 36 being for example an LPE type sub-program.

If only one seed voxel 31 is chosen, the computation by the microprocessor 11 will take some time. This processing can be accelerated by designating several seed voxels. For example it is possible to choose two seed voxels located in the region 29 of the lithiasis. The view 26 of the section 27 will be a segmented view relative to a threshold 37. The threshold 37 will be a threshold with a high value that is well known as being representative of lithiasis, for example above 900 in standardized Hounsfield units or HU. The voxel chosen is then chosen in the region of definite lithiasis. If several seed voxels have to be chosen, in addition to the voxel 31 it is then possible to choose a voxel 38 located in the zone 28 so that the voxels 31 and 38 are placed in two regions of the image thus segmented. Naturally, it is possible in each region to choose several voxels so as to further accelerate the gradient computation.

It can be seen that this manner of proceeding is appreciably less cumbersome than the former process because, firstly, it can be arbitrary (voxel 24) and, secondly, if it is assisted by the operator by the choice of the voxel 31, it requires the display of only one view 26. Furthermore, and above all, it has no effect on the result of the computation. Thus, an embodiment of the method of the invention is independent of the place at which the practitioner places the marker, the seed voxel, in the lithiasis. It affects only the speed with which the computation is performed. However, the determining of the envelope of the places 30 will always be the same.

To further accelerate the computation, it can be seen to it that the computation of the gradient is done on macro-voxels 39, for example groupings of 27 adjacent voxels or groupings of 729 (27 x 27) adjacent voxels or other groupings. It can be planned that, once the maximum of the gradient 30 has been found for such a macro-voxel 39, the computation will be oversampled and performed for elementary voxels such as the voxel 40 corresponding to this macro-voxel 39. This mode of action accelerates the computation. In this case it is possible, for the constitution of the volume image 33 to decide on the assigning, to all the voxels of a macro-voxel that does not contain the maximum of the gradient, of the gradient value measured for the macro-voxel. It is also possible to include an over-sampling of the data before processing to improve the precision of the computation.

It may be planned to have a classic type of noise-elimination filtering operation during the measurement of the function of the sub-program 18, and the reading of the projected images 19 to 21. Similarly, at the time of the display of the views 26 or 34 it may be planned to have a display filtering, a smoothing, so that these images appear in a more pleasant way.

Furthermore, to make the results even more easily exploitable by a practitioner, measure a volume V of the lithiasis. Simply, this volume V may be represented by the number of voxels located in the envelope determined by the maximum of the gradient, in the envelope determined by the contours 30. Thus, a volume is obtained. It will be noted that the value of this volume is directly proportional to the number of voxels contained in the lithiasis. In Figure 3, in one improvement, shows a weighted volume in which each voxel comes into play proportionally to the value of the measurement of its radiological density. The graph of Figure 3 is an enlargement of the equivalent of the first graph of Figure 2. It thus shows that, between a lower threshold value v1 and another threshold value v2 of radiological density, at the position 29 of the lithiasis, the voxels i may be taken into consideration and the function of the ratio between, firstly, the difference in their radiological density vi-v1 and, secondly, the height of the expansion of radiological density v2-v1. This taking of voxels into consideration is done at least for the voxels for which the value is below v2. As a variant, v2 is a maximum value of radiological density in the lithiasis. Thus, the volume V will be expressed, for example, in Equation (1) in the form of: V = Σ (vi - v1)/(v2 - v1).

Through this action, it is realized that there is an ability to differentiate between stones that might have the same envelope volume but different profiles of evolution of radiological density. To this end, as is shown in the second graph in Figure 2, it is decided to choose a maximum G of a gradient of the radiological density signal only if this value G is itself higher than a threshold g, at least a noise threshold. In one example, G is such that g = 1/3 G. Allowing for a smaller gradient quite simply means that the slope 41 of the radiological density signal is less steep and therefore that a zone 42 of imprecision on the position of the exact boundary of the envelope 30 may be more or less wide. In an embodiment of the invention, to avoid having to accept the randomness of this imprecision, in the measurement of the volume, consider the entire zone 42, each voxel being taken into consideration proportionately to the ratio referred to here above.

Through this mode of action, it is possible to have a direct volume, namely the one contained in the contour 30, and also preferably have a weighted volume, that is far more exact. From one time to the next, for example after an interval of several weeks, the practitioner can measure the effects of the treatment applied to this volume or to this weighted volume as a function of the treatment he or she has proposed to the object body, and independently of the form of the images seen on the screen.

The above characterization represents the span, or even the weighted span, of the lithiasis. It does not represent its strength. In this case, to give a more exact measurement of this strength, the mean density D is computed in Equation (2) in the form: D = Σ vi (vi - v1)/(v2 - v1). This computation of the mean density D may be standardized or not standardized by the volume V measured by the previous Equation (1). The voxels whose value is below v1 are set aside. If necessary, with the relative value of each voxel of the image being known, it is possible to plot a histogram of the population of candidate voxels. In this population, it is possible, rather than give a value of mean density, to give a value of density of the first quartile or third quartile, or even a standard deviation value. All these elements are attributes that may be associated in the image 23 or the image 33 and subsequently enable the images to be compared with one another.

Equation (1) is computed by means of a sub-program 43 of the program 13 while the mean density is computed by means of a sub-program 44 of the program 13.

Figure 4 furthermore gives the view, by way of an improvement, of a segmentation of the image of radiological density as a function of at least three thresholds s1, s2, and s3. In one example, the threshold s1 is a bottom threshold substantially equal to 300 Hounsfield units HU. The threshold s2 is set at around 400 HU, the threshold s3 being set for example at 1125 HU. The value of the threshold s1 corresponds to a standardized value of bone. The calcifications will be qualified, so to speak, by their greater density relative to the object body's bone. The thresholds s1, s2 and s3 are themselves adjustable by the practitioner using ergonomic commands placed at his disposal with the peripheral 15.

The thresholds s1 to s3 are used to determine four classes c1 to c4 in the object represented. For the voxels concerned by one class, assign them one color in the section views such as 26. For example the class c4 is assigned the color red, the class, c3 is assigned the color emerald-green, the class c2 is assigned the color yellow and the class c1 is assigned the color pale blue. For a given display of an image of a patient, the thresholds s1, s2 and s3 are stored as being display parameters. In images obtained several weeks later, the same parameters can be applied to compare the images with one another, and quickly find out if the recommended therapy has had sufficient effect or not. Naturally, in addition to this comparison with color codes, it is advisable to make comparisons of volumes and densities from one time to another. Furthermore the number of thresholds and classes may be modified.

Since all the processing operations are automatic, inasmuch as the images are assigned to a same object body, for example by an administrative reference assigned to a file for the storage of the digital volumes or images, it becomes possible to make routine comparisons between the different variables measured, namely volume, weighted volume, mean density, standard deviation or other variables. These comparisons can be represented on a graph having the values of these variables on the y-axis and the date on which these values have been measured on the x-axis. The automatic report is therefore made by integrating all the measurements, screenshots, statistics in HU units, size, etc. resulting from the post-processing. This is done automatically in a report in portable form, for example of the pdf or .rtf type. The practitioner may retrieve the corresponding files on his microcomputer for revision of necessary and send them on to another practitioner, for example a prescribing urologist or a surgeon. In the prior art, on the contrary, the practitioner makes prints on film and dictates a report. A secretary then types this report out. The automatic report provides for screen images and all the other measurements in digital and subsequently editable form.

In practice, the lower threshold s1 is below a value of radiological density for which a maximum of the gradient of radiological density is measured. Typically s1 is below the value of radiological density at the position of the contour 30. In practice, s1 is chosen to be equal to 300 HU that corresponds to the radiological density of bone. In this case, apart from the lithiasis, all the rest of the image becomes pale blue.

Figure 1 showed that, during the radiological examination, the object body might be subjected to an injection of a contrast medium 45. Thus, during the examination, several images of the type 19 to 21 will be taken, these images themselves leading to several images of the type 23. A first 23 type image is a non-contrasted image made before the injection of the contrast product. A second image and a third image correspond to nephrographic and pyelolographic states which themselves correspond to a diffusion and a metabolization of the product injected into the object's body 1. These images are then also compared with one another to refine the measurement of the lithiasis.

In the context of operations with surgical tools, it is far easier to determine a path from an entry point and display the anatomy being traveled through in sections transversal to this path. Indeed, this anatomy is far better identified and far better segmented.

For completeness, various aspects of the invention are set out in the following numbered clauses:
1. A method for the preparation of a renal image comprising:
   subjecting a body with a kidney to a sequence of irradiation at various angles;
      taking images in projection at each of these angles;
   forming a first 3D image by voxels produced from the images in projection, each voxel being assigned a measurement of the radiological density at its position, the first image representing the body in and around the kidney;
   choosing a seed voxel;
   exploring from the seed voxel the neighboring voxels in the first image;
   measuring a gradient of the radiological density for all the explored voxels;
      selecting the voxels located on an envelope for which the gradient is the maximum and thus obtaining an envelope surface of a renal affection; and
   producing a second image representing the envelope and visualizing the second image.
2. The method according to clause 1 comprising:
   when chosing a seed voxel, a segmentation of the first image is made with a high threshold value;
   viewing the first segmentation image thus segmented; and
   designating a seed voxel in the first segmented image, the seed voxel being a voxel situated in a region of the image where the radiological density measured is higher than the threshold.
3. The method according to one of the clauses 1 to 2 comprising:
   choosing several seed voxels distributed in the first image.
4. The method according to clause 3 comprising:
   a segmentation of the first image is made with a threshold value; and
      seed voxels placed in two regions, thus segmented, of the image are chosen.
5. The method according to one of the clauses 1 to 4 comprising:
   selecting the voxels for which the gradient of the radiological density is the maximum;
   oversampling the voxels situated in the first image, in an environment close to the selected voxels; and
   specifying the selection on these oversampled voxels.
6. The method according to one of the clauses 1 to 5 comprising:
   selecting a first filtered image and/or a second filtered image.
7. The method according to one of the clauses 1 to 6 comprising:
   computing a volume of the envelope in the second image; and
   presenting the result of the computation.
8. The method according to clause 7 comprising:
   computing a weighted volume of the envelope in the second image.
9. The method according to one of the clauses 1 to 8 comprising:
   computing a mean value of the radiological density in the envelope; presenting the result of the computation.
10. The method according to one of the clauses 1 to 9 comprising:
   producing and comparing second images separated in time; and
   comparing the computed volumes of the envelopes.
11. The method according to one of the clauses 1 to 10 comprising:
   producing second images, corresponding to a state of the body before and after injection of contrast media; and
   comparing the second images.
12. The method according to one of the clauses 1 to 11 comprising:
   choosing at least three thresholds of radiological density;
   assigning one class to each voxel among a plurality of classes corresponding to a range of radiological density between two thresholds in which the information that they contain is situated; and
   displaying the voxels by assigning them different colors, in the displayed image, according to their class.
13. The method according to clause 12 comprising:
   determining a low threshold among the thresholds chosen; and
   the low threshold being lower than a value of radiological density for which a maximum of the gradient of radiological density is measured.
14. The method according to one of the clauses 1 to 13 wherein the thresholds are made to vary.
15. The method according to one of the clauses 1 to 14 comprising:
   choosing a gradient value if it is above a threshold.
16. The method according to one of the clauses 1 to 15 comprising:
   selecting the voxels by a watershed algorithm.
17. An apparatus comprising:
   means (3) for provided a source of radiation to move about an axis of rotation (5) to irradiate at various angles (7,8, 9) a body (1) having a kidney (2);
   means (4) for detecting the radiation after passing through the body;
   means (10, 11, 12, 13, 14, 15, 17, 18, 22) for controlling operation of the apparatus;
   means (16) for displaying images (19, 20, 21) taken in projection at the various angles;
   the means for controlling comprising means for forming a first 3D image by voxels produced from the images in projection, each voxel being assigned a measurement of the radiological density at its position, the first image representing the body in and around the kidney;
   choosing a seed voxel;
   exploring from the seed voxel the neighboring voxels in the first image;
   measuring a gradient of the radiological density for all the explored voxels;
   selecting the voxels located on an envelope for which the gradient is the maximum and thus obtaining an envelope surface of a renal affection; and
   producing a second image representing the envelope and visualizing the second image.
18. An apparatus comprising means for carrying out the method of any one of clauses 1 to 16.
19. A computer program comprising program code means for implementing the steps of the method according to any one of clauses 1 to 16, when the program runs on a computer.
20. A computer program product comprising a computer useable medium having computer readable program code means embodied in the medium, the computer readable program code means implementing the steps of the method according to any one of clauses 1 to 16.
21. An article of manufacture for use with a computer system, the article of manufacture comprising a computer readable medium having computer -readable program code means embodied in the medium, the program code means implementing the steps of the method according to any one of clauses 1 to 16.
22. A program storage device readable by a machine tangibly embodying a program of instructions executable by the machine to perform the steps of the method according to any one of clauses 1 to 16.

## Claims

1. A method for the preparation of a renal image comprising:
subjecting a body with a kidney to a sequence of irradiation at various angles;
taking images in projection at each of these angles;
forming a first 3D image by voxels produced from the images in projection, each voxel being assigned a measurement of the radiological density at its position, the first image representing the body in and around the kidney;
choosing a seed voxel;
exploring from the seed voxel the neighboring voxels in the first image;
measuring a gradient of the radiological density for all the explored voxels;
selecting the voxels located on an envelope for which the gradient is the maximum and thus obtaining an envelope surface of a renal affection; and
producing a second image representing the envelope and visualizing the second image.

2. The method according to claim 1 comprising:
when chosing a seed voxel, a segmentation of the first image is made with a high threshold value;
viewing the first segmentation image thus segmented; and
designating a seed voxel in the first segmented image, the seed voxel being a voxel situated in a region of the image where the radiological density measured is higher than the threshold.

3. The method according to one of the claims 1 to 2 comprising:
choosing several seed voxels distributed in the first image.

4. The method according to claim 3 comprising:
a segmentation of the first image is made with a threshold value; and
seed voxels placed in two regions, thus segmented, of the image are chosen.

5. The method according to one of the claims 1 to 4 comprising:
selecting the voxels for which the gradient of the radiological density is the maximum;
oversampling the voxels situated in the first image, in an environment close to the selected voxels; and
specifying the selection on these oversampled voxels.

6. The method according to one of the claims 1 to 5 comprising:
computing a mean value of the radiological density in the envelope; presenting the result of the computation.

7. The method according to one of the claims 1 to 6 comprising:
producing and comparing second images separated in time; and
comparing the computed volumes of the envelopes.

8. The method according to one of the claims 1 to 7 comprising:
choosing at least three thresholds of radiological density;
assigning one class to each voxel among a plurality of classes corresponding to a range of radiological density between two thresholds in which the information that they contain is situated; and
displaying the voxels by assigning them different colors, in the displayed image, according to their class.

9. An apparatus comprising:
means (3) for provided a source of radiation to move about an axis of rotation (5) to irradiate at various angles (7,8, 9) a body (1) having a kidney (2);
means (4) for detecting the radiation after passing through the body;
means (10, 11, 12, 13, 14, 15, 17, 18, 22) for controlling operation of the apparatus;
means (16) for displaying images (19, 20, 21) taken in projection at the various angles;
the means for controlling comprising means for forming a first 3D image by voxels produced from the images in projection, each voxel being assigned a measurement of the radiological density at its position, the first image representing the body in and around the kidney;
choosing a seed voxel;
exploring from the seed voxel the neighboring voxels in the first image;
measuring a gradient of the radiological density for all the explored voxels;
selecting the voxels located on an envelope for which the gradient is the maximum and thus obtaining an envelope surface of a renal affection; and
producing a second image representing the envelope and visualizing the second image.

10. A computer program comprising program code means for implementing the steps of the method according to any one of claims 1 to 8, when the program runs on a computer.
